# EUROPÄISCHE PATENTANMELDUNG

(11) **EP 3 604 493 A1**
(43) Veröffentlichungstag der Anmeldung: **05.02.2020**
(21) Anmeldenummer: 19188925.2
(22) Anmeldetag: 29.07.2019
(51) Int. Cl.: C12M 1/107, C12M 1/00, C12M 1/06

(54) **SCHLIESSVORRICHTUNG FÜR FERMENTIERBEHÄLTER**

(30) Priorität: 03.08.2018 DE 102018118870; 05.10.2018 DE 102018124609
(71) Anmelder: Thürwächter GmbH & Co. KG, 87477 Sulzberg (DE)
(72) Erfinder: Thürwächter, Paul, 87477 Sulzberg (DE)
(74) Vertreter: Patentanwälte Olbricht, Buchold, Keulertz Partnerschaft mbB

(57) **Zusammenfassung**

Die Erfindung betrifft eine Vorrichtung zum Öffnen und Schließen einer in einer Deckwand eines Fermentierbehälters vorgesehenen Wartungsöffnung mit einem Verschlusselement zum wahlweisen Öffnen und Schließen der Wartungsöffnung wobei eine Trennwand zum gasdichten Abtrennen eines Volumens unterhalb der Wartungsöffnung vorgesehen ist. Die Erfindung betrifft weiterhin eine Biogasanlage mit einer Wartungsöffnung und ein Verfahren zum Öffnen eines Verschlusselementes einer Biogasanlage.

## Beschreibung

Die Erfindung betrifft eine Vorrichtung zum Öffnen und Schließen einer in einer Deckwand eines Fermentierbehälters vorgesehenen Wartungsöffnung, mit einem Körper zum Verbinden der Vorrichtung mit einer Wartungsöffnung eines Fermentierbehälters und einem Verschlusselement zum wahlweisen Öffnen und Schließen der Wartungsöffnung. Ferner betrifft die Erfindung eine Biogasanlage und ein Verfahren zum Öffnen und Schließen einer in einer Deckwand eines Fermentierbehälters vorgesehenen Wartungsöffnung.

Fermentierbehälter dienen in Biogasanlagen dazu, ein in dem Fermentierbehälter angeordnetes überwiegend flüssiges Substrat zu fermentieren. Das Substrat kann beispielsweise Exkremente von Nutzvieh und pflanzliche Abfälle enthalten.

Ein bei der Fermentation des Substrats gebildetes Biogas steigt aus dem Substrat nach oben und reichert sich in einem Volumen des Fermentierbehälters oberhalb eines Flüssigkeitsspiegels des Substrats an. Zum Ableiten des Biogases aus dem Ferientierbehälter ist eine Fluidleitung vorgesehen, welche mit dem das Biogas enthaltenden Volumen des Fermentierbehälters fluidverbunden ist. Das auf diese Weise gewonnene Biogas kann beispielsweise zum Antreiben eines Fahrzeugs oder zum Erzeugen einer elektrischen Energie verwendet werden.

Um den Wirkungsgrad der Fermentation zu erhöhen, umfassen Biogasanlagen gewöhnlich Rühreinheiten mit Propellern. Die Propeller sind in dem Substrat unterhalb des Flüssigkeitsspiegels angeordnet und können durch Rotieren das Substrat kontinuierlich oder in zeitlichen Intervallen bewegen und durchmischen, wodurch der Fermentation abträgliche Inhomogenitäten des Substrats, insbesondere einer Bildung von Schichten in dem Substrat entgegengewirkt wird.

Allerdings muss eine solche Rühreinheit, die auch als Rührwerk bezeichnet wird, regelmäßig gewartet und im Falle eines Defektes auch repariert oder ausgetauscht werden. Dies ist praktisch ausgeschlossen, wenn die Rühreinheit in dem Substrat angeordnet ist. Deshalb sind in einer Deckwand des Fermentierbehälters eine Wartungsöffnung und in dem Fermentierbehälter eine sich durch die Wartungsöffnung erstreckende Säule vorgesehen, an der die Rühreinheit mittels einer Linearführung verschiebbar gehalten ist. Zum Zwecke der Wartung oder Reparatur kann die Rühreinheit entlang der Säule an eine Außenseite des Fermentierbehälters verfahren werden, wo sie für Fachpersonal einfach zugänglich ist.

Während des normalen Betriebs der Biogasanlage ist die Wartungsöffnung durch eine entsprechende Vorrichtung geschlossen, damit das Biogas nicht unkontrolliert durch die Wartungsöffnung austreten kann. Zum Warten oder Reparieren der Rühreinheit muss die Wartungsöffnung dagegen freigegeben werden, so dass das Biogas ungenutzt durch die Wartungsöffnung nach außen entweicht. Zum einen geht bereits gebildetes Biogas auf diese Weise verloren, zum anderen ist das gebildete Biogas leicht entzündlich, was mit einer entsprechenden Brand- und Explosionsgefahr außerhalb des Fermentierbehälters einhergeht. Abgesehen davon belastet das Biogas wie ein Treibgas die Umwelt.

Es ist daher eine Aufgabe der vorliegenden Erfindung, eine Vorrichtung der eingangs genannten Art zu schaffen, die bei einer Wartung oder Reparatur der Rühreinheit einem Entweichen des Biogases entgegenwirkt.

Gegenstand der Erfindung ist eine Vorrichtung zum Öffnen und Schließen einer in einer Deckwand eines Fermentierbehälters vorgesehenen Wartungsöffnung, mit einem Körper zum Verbinden der Vorrichtung mit einer Wartungsöffnung eines Fermentierbehälters und einem Verschlusselement zum wahlweisen Öffnen und Schließen der Wartungsöffnung. Mit anderen Worten schlägt die Erfindung eine Vorrichtung vor, welche ein Öffnen und Schließen der Wartungsöffnung mit einem Verschlusselement erlaubt.

Erfindungsgemäß umfasst die Vorrichtung eine Trennwand zum Abtrennen eines Volumens unterhalb der Wartungsöffnung und einen Betätigungsmechanismus zum wahlweisen Absenken und Anheben der Trennwand zwischen einer angehobenen Ruhestellung und einer abgesenkten Trennstellung. Die Trennwand ist demnach ausgebildet, wahlweise ein Volumen im Inneren des Fermentierbehälters abzutrennen. Dazu ist sie aus einer Ruhestellung, in der sie kein Volumen abtrennt, in eine Trennstellung, in der sie das Volumen abtrennt, absenkbar oder aus der Trennstellung in die Ruhestellung anhebbar.

Erfindungsgemäß ist ein an der Vorrichtung, insbesondere an dem Körper vorgesehener Verriegelungsmechanismus zum Verriegeln und Entriegeln des Verschlusselements in einer Schließstellung des Verschlusselements vorgesehen. Durch den Verriegelungsmechanismus kann sichergestellt werden, dass das Verschlusselement nicht versehentlich aus seiner Schließstellung fortbewegt wird. Ein Fortbewegen des verriegelten Verschlusselements ist ausgeschlossen. Lediglich das entriegelte Verschlusselement kann aus der Schließstellung fortbewegt werden.

Weiterhin ist erfindungsgemäß vorgesehen, dass der Verriegelungsmechanismus derart ausgebildet ist, dass das Verschlusselement nur in der abgesenkten Trennstellung der Trennwand verriegelbar und/oder entriegelbar ist. Infolgedessen kann der Verriegelungsmechanismus nicht betätigt werden, wenn die Trennwand in einer anderen als der abgesenkten Trennstellung ist. Dadurch werden Fehlbedienungen unmöglich gemacht und die Arbeitssicherheit an der Vorrichtung signifikant verbessert.

Bei einer bevorzugten Ausgestaltung des Vorschlags ist vorgesehen, dass die Trennwand zum gasdichten Verbinden eines in dem Inneren des Fermentierbehälters angeordneten Flüssigkeitsspiegels mit dem Körper ausgebildet ist. Entsprechend kann die Trennwand derart abgesenkt werden, dass sie in ein in dem Fermentierbehälter vorhandenes überwiegend flüssiges Substrat eingetaucht ist.

Geschickter Weise ist vorgesehen, dass der Verriegelungsmechanismus einen an dem Körper vorgesehenen Durchlass, einen an dem Verschlusselement vorgesehenen Durchlass, die in der Schließstellung des Verschlusselements miteinander fluchten, und eine Welle umfasst, wobei die Welle die beiden Durchlässe in einem verriegelten Zustand des Verriegelungsmechanismus durchgreift und in einem entriegelten Zustand des Verriegelungsmechanismus aus den beiden Durchlässen herausgezogen ist. Mit anderen Worten wirkt die Welle als ein Riegel, der zum Verriegeln durch die beiden Durchlässe gesteckt wird und zum Entriegeln aus diesen entfernt wird.

Des Weiteren ist günstiger Weise vorgesehen, dass der Verriegelungsmechanismus eine an der Welle befestigte Seiltrommel und ein mit der Seiltrommel verbundenes Seil umfasst. Mittels des Seils lässt sich die Welle mit einer Kraft beaufschlagen, um die Welle in den beiden Durchlässen festzulegen und ein Entriegeln auszuschließen.

Vorteilhafter Weise ist vorgesehen, dass die Welle im verriegelten Zustand einen zum Absenken und Anheben der Trennwand vorgesehenen Abschnitt des Seils trägt. Der zum Bewegen der Trennwand vorgesehene Abschnitt kann in der Seiltrommel aufgerollt sein. Wenn die Trennwand nicht in ihrer Trennstellung ist, ist das Seil gespannt und die Welle mit einer Gewichtskraft der Trennwand beaufschlagt. Wenn dagegen die Trennwand in ihrer Trennstellung ist, ist das Seil entspannt und die Welle nicht mit der Gewichtskraft beaufschlagt. Aufgrund dessen lässt sich die Welle ausschließlich in der Trennstellung aus den Durchlässen herausziehen, weshalb ein Entriegeln des Verschlusselements nur in der Trennstellung möglich ist.

Geschickter Weise ist vorgesehen, dass der Betätigungsmechanismus derart ausgebildet und angeordnet ist, dass die Trennwand auf einer der Trennwand gegenüberliegenden Seite des Körpers betätigbar ist. Ein solcher Betätigungsmechanismus erlaubt ein komfortables Betätigen der Trennwand außerhalb des Fermentierbehälters.

In einer vorteilhaften Ausgestaltung ist vorgesehen, dass der Betätigungsmechanismus die in den beiden Durchlässen drehbar gelagerte Welle und die mit der Welle drehfest verbundene Seiltrommel zum wahlweisen Aufrollen und Abrollen des Seils umfasst. Damit haben die Seiltrommel und das Seil eine Doppelfunktion. Sie wirken nicht nur innerhalb des Verriegelungsmechanismus, sondern können auch zum Bewegen der Trennwand verwendet werden.

In weiteren Ausgestaltungen sind ein erstes freies Ende des Seils mit der Seiltrommel und ein zweites freies Ende des Seils mit einem freien Endbereich der Trennwand verbunden. Entsprechend ist das Seil in der Trennstellung der Trennwand von der Seiltrommel im Wesentlichen vollständig abgerollt und in der Ruhestellung der Trennwand in der Seiltrommel im Wesentlichen aufgerollt.

In einer weiteren bevorzugten Ausführungsform ist vorgesehen, dass der Betätigungsmechanismus und/oder der Verriegelungsmechanismus zwei Seiltrommeln, die an gegenüberliegenden freien Enden der Welle angeordnet sind, und zwei Seile umfasst, die mit gegenüberliegenden freien Endbereichen der Trennwand verbunden sind. Mittels zweier Seile lässt sich die Trennwand symmetrisch mit einer Kraft beaufschlagen, wodurch eine Gefahr eines Verkantens der Trennwand während des Absenkens oder Anhebens verringert wird.

Des Weiteren ist günstiger Weise vorgesehen, dass der Betätigungsmechanismus eine Antriebseinheit zum drehenden Antreiben der Welle aufweist. Die Antriebseinheit erlaubt ein erleichtertes Drehen der Welle.

Vorteilhafterweise umfasst die Antriebseinheit eine mit der Seiltrommel lösbar verbindbare Kurbel, insbesondere eine gebremste Ratschenkurbel. Die Kurbel bildet eine in der Handhabung besonders einfache Antriebseinheit für ein manuelles Bewegen der Trennwand. Sie kann zum Bewegen der Trennwand mit der Welle drehfest verbunden werden und nach einem Bewegen der Trennwand von der Welle gelöst werden. Eine gebremste, d.h. unidirektional verrastende Ratschenkurbel kann die zum Aufrollen des Seils erforderliche Kraft verringern.

Geschickter Weise ist vorgesehen, dass der Betätigungsmechanismus zu einem, insbesondere zu jedem Seil eine an dem Körper drehbar gelagerte Umlenkrolle zum Führen des Seils umfasst. Dank der Umlenkrolle ist die Flexibilität bei der Führung des Seils vergrößert.

In einer Ausführungsform erstreckt sich jedes Seil durch eine in dem Körper vorgesehene Seilöffnung. Die Seilöffnung ist ein einfaches Mittel, um das Seil von der der Trennwand gegenüberliegenden Seite des Körpers auf die der Trennwand zugewandten Seite des Körpers zu führen.

Vorteilhaft kann die Trennwand einen Kunststoff umfassen oder aus einem Kunststoff bestehen. Kunststoffe stehen in einer großen Vielfalt zur Verfügung und sind bei der Herstellung einfach zu handhaben.

Alternativ oder zusätzlich kann die Trennwand ein Gewebe umfassen oder aus einen Gewebe bestehen. Gewebe können eine leichte Biegbarkeit und eine starke Zugfestigkeit aufweisen.

Bei einer bevorzugten Ausgestaltung des Vorschlags ist vorgesehen, dass die Trennwand eine schlauchförmige Schürze umfasst. Die Schlauchform umschließt in der Trennstellung der Trennwand einen relativ kleines mit der Wartungsöffnung fluidverbundenes Volumen und ggf. eine sich durch das Volumen aus dem Inneren des Fermentierbehälters durch die Wartungsöffnung nach außen erstreckende Säule. Ein in diesem von der Trennwand umschlossenen Volumen vorhandenes Biogas kann in der Offenstellung des Verschlusselements unkontrolliert durch die Wartungsöffnung nach außen entweichen. Allerdings ist das relativ größere Volumen an der Außenseite der Trennwand von der Wartungsöffnung gasdicht getrennt, so dass das dort angeordnete Biogas nicht unkontrolliert durch die Wartungsöffnung nach außen entweichen kann und daher während einer Wartung oder Reparatur der Rühreinheit in dem Fermentierbehälter verbleibt.

Vorteilhaft ist die Schürze bzw. die Trennwand ziehharmonikaartig faltbar ausgebildet. Dadurch ist das Absenken und Anheben der Trennwand erleichtert, und die Trennwand benötigt in der Ruhestellung wenig Raum.

Geschickter Weise ist vorgesehen, dass die Trennwand einen starren Rahmen umfasst, der insbesondere an einem freien Endbereich der Trennwand angeordnet und mit diesem verbunden ist. Der Rahmen stabilisiert die Trennwand und definiert entsprechend seiner Form eine Querschnittsfläche der Trennwand.

Bei weiteren Ausgestaltungen ist der Rahmen an einem dem Körper zugewandten Endbereich und/oder an einem dem Körper abgewandten Endbereich der Trennwand angeordnet. Bei dieser Anordnung sorgt der Rahmen für eine in der Trennstellung der Trennwand über die gesamte Länge der Trennwand im Wesentlichen konstante Querschnittsfläche.

Alternativ kann die Trennwand mehrere Wandelemente umfassen. Auch die aus mehreren Wandelementen gebildete Trennwand kann durch Verschieben der Wandelemente in ihrer Länge geändert werden, um sie zwischen der Ruhestellung und der Trennstellung zu bewegen.

Bei dieser Ausgestaltung können die Wandelemente relativ zueinander teleskopartig verschiebbar ausgebildet und angeordnet sein. Auf diese Weise lässt sich eine bewährte und einfache Längenvariabilität der Trennwand schaffen.

Bevorzugt ist die Trennwand an dem Körper befestigt. Die Befestigung an dem Körper verhindert, dass Biogas zwischen dem Körper und der Trennwand unkontrolliert nach außen entweichen kann. Zudem trägt der Körper die Trennwand in der Trennstellung, wodurch das Seil entlastet und das Abschlusselement entriegelbar ist.

Bei vorteilhaften Ausgestaltungen weist die Trennwand eine Sonde, insbesondere zum Erfassen eines elektrischen Widerstands auf. Der elektrische Widerstand einer Strecke innerhalb des Substrats ist geringer als der elektrische Widerstand derselben Strecke außerhalb des Substrats, d.h. innerhalb des über Flüssigkeitsspiegel des Substrats angeordneten Biogases. Deshalb kann durch eine Messung des elektrischen Widerstands die vertikale Position des Flüssigkeitsspiegels bestimmt werden. Es eignen sich aber auch andere Sonden, die in der Lage sind, die Anwesenheit des Flüssigkeitsspiegels zu entdecken, z.B. kapazitive oder auch optische Sensoren.

Geschickter Weise ist vorgesehen, dass die Sonde an einem dem Körper abgewandten Endbereich der Trennwand angeordnet ist und/oder stabförmig ausgebildet ist und sich insbesondere von dem Körper weg weisend erstreckt. Mit anderen Worten lässt sich auf diese Weise feststellen, ob die Trennwand bis zu dem Substrat reicht oder in das Substrat eingetaucht ist, also den gesamten Abstand zwischen der Wartungsöffnung und dem Flüssigkeitsspiegel überbrückt. Wenn eine weitere derartige Sonde zusätzlich an der Rühreinheit angebracht ist, lässt sich schaltungstechnisch vorsehen, dass die Rühreinheit nur betrieben werden kann, wenn die Rühreinheit unterhalb des Flüssigkeitsspiegels und das freie Ende der Trennwand oberhalb des Flüssigkeitsspiegels angeordnet ist. Infolge dieser räumlichen Trennung von Rühreinheit und Trennwand ist gewährleistet, dass die Trennwand während des Betriebs der Rühreinheit weder durch die Rühreinheit selbst noch durch das von ihr bewegte Substrat beschädigt wird.

In einer weiteren bevorzugten Ausführungsform ist vorgesehen, dass das Verschlusselement eine Platte umfasst. Platten sind einfach in der Herstellung und Handhabung. Beispielsweise kann das Verschlusselement eine aus einem Metall gefertigte Platte umfassen.

Vorteilhaft weist die Platte zwei Plattenabschnitte auf, die sich insbesondere senkrecht zueinander erstrecken und/oder an einer gemeinsamen Kante miteinander verbunden sind. Mit einer derart ausgebildeten Platte lassen sich komplexe Öffnungsflächen verschließen.

Geschickter Weise ist vorgesehen, dass in der Schließstellung des Verschlusselements ein Plattenabschnitt einen kastenförmigen Aufbau des Körpers seitlich verschließt. Der kastenförmige Aufbau kann eine außerhalb der Wartungsöffnung angeordnete Komponente der Biogasanlage zumindest teilweise einhausen.

Bei weiteren Ausführungsformen weist das Verschlusselement ein längliches Verstärkungsprofil auf, das mit einem Plattenabschnitt lösbar verbunden, insbesondere verschraubt ist. Das Verstärkungsprofil dient der stabilen Befestigung des Verschlusselements.

Vorteilhaft weist das Verschlusselement zwei oder mehr als zwei Verstärkungsprofile auf, die sich insbesondere parallel zueinander und/oder jeweils parallel zu dem Plattenabschnitt erstrecken. Dadurch wird die Stabilität der Befestigung weiter verbessert.

Des Weiteren ist bevorzugt, dass das Verschlusselement an der Vorrichtung demontierbar oder drehbar gelagert angeordnet ist. Mit anderen Worten lässt sich das Verschlusselement von der Vorrichtung lösen oder gegenüber der Vorrichtung verdrehen.

Das Verschlusselement kann mit dem Körper lösbar verbunden oder an dem Körper zwischen der Schließstellung und einer Offenstellung des Verschlusselements verschwenkbar gehalten sein. Damit ist das Verschlusselement in der Offenstellung von dem Körper entfernt oder gegenüber dem Körper verschwenkt.

Geschickter Weise ist die Vorrichtung in die Wartungsöffnung einsetzbar. Eine Außenkontur der Vorrichtung korrespondiert dazu mit einer Innenkontur der Wartungsöffnung. Infolgedessen bedarf es keiner baulichen Veränderung des Fermentierbehälters, um die Vorrichtung zu verwenden.

Des Weiteren ist günstiger Weise vorgesehen, dass der Körper einen mit einem Randbereich der Wartungsöffnung verbindbaren, insbesondere verschraubbaren geschlossenen Rahmen umfasst. Im bestimmungsgemäßen Zustand bildet der Rahmen einen die Wartungsöffnung umgebenden Übergangsbereich zwischen der Vorrichtung und dem Fermentierbehälter.

Vorteilhaft weist der Rahmen eine umlaufende erste Anlagefläche auf, die im bestimmungsgemäß montierten Zustand der Vorrichtung an dem Randbereich der Wartungsöffnung anliegt. Die Anlagefläche stellt sicher, dass die Vorrichtung gasdicht an den Randbereich der Wartungsöffnung anschließt.

Ebenso vorteilhaft weist der Rahmen eine umlaufende zweite Anlagefläche auf, an der das Verschlusselement in der Schließstellung des Verschlusselements anliegt. Die zweite Anlagefläche stellt sicher, dass das Verschlusselement gasdicht an dem Körper der Vorrichtung anliegt.

In weiteren Ausgestaltungen weist der Rahmen eine Mehrzahl von flachen Verbindungsstreben auf, die sich insbesondere senkrecht zwischen der ersten Anlagefläche und der zweiten Anlagefläche erstrecken und/oder eine trapezförmige Außenkontur aufweisen. Die Verbindungsstreben verstärken den Rahmen und verringern ein Verformen des Rahmens unter Krafteinwirkung.

Gegenstand der Erfindung ist auch eine Biogasanlage, umfassend einen Fermentierbehälter mit einer in einer Deckwand des Fermentierbehälters ausgebildeten Wartungsöffnung. Solche Biogasanlagen sind weit verbreitet, wodurch sich für die vorliegende Erfindung eine Vielzahl von Verwendungsmöglichkeiten ergibt.

Erfindungsgemäß umfasst die Biogasanlage eine erfindungsgemäße Vorrichtung und/oder eine Vorrichtung, die in einem erfindungsgemäßen Verfahren bedient wird. Damit ist die Biogasanlage vor einem unkontrollierten Entweichen einer überwiegenden Menge eines in dem Fermentierbehälter vorhandenen Biogases nach außen geschützt.

In weiteren Ausgestaltungen umfasst die Biogasanlage eine Rühreinheit. Die Rühreinheit dient der Homogenisierung eines in der Biogasanlage angeordneten im Wesentlichen flüssigen Substrats und wirkt daher einer unerwünschten Bildung von Schichten in dem Substrat entgegen.

Ein weiterer Gegenstand der Erfindung ist ein Verfahren zum Öffnen eines Verschlusselements für einen Fermentierbehälter einer erfindungsgemäßen Biogasanlage, wobei ein Öffnen des Verschlusselements erst erfolgt, wenn die Trennwand abgesenkt ist. Bei abgesenkter Trennwand ist zumindest ein Teil eines in dem Fermentierbehälter vorhandenen Biogases an einem unkontrollierten Entweichen durch das geöffnete Verschlusselement nach außen gehindert.

Vorteilhaft ist vorgesehen, dass ein Öffnen des Verschlusselements erst erfolgt, wenn die Vorrichtung durch die Trennwand gasdicht mit dem Flüssigkeitsspiegel im Inneren des Fermentierbehälters verbunden ist. In dieser Stellung der Trennwand ist ein Volumen innerhalb des Fermentierbehälters von der Wartungsöffnung gasdicht getrennt.

Noch ein Gegenstand der vorliegenden Erfindung ist ein Verfahren zum Schließen eines Verschlusselements für einen Fermentierbehälter einer erfindungsgemäßen Biogasanlage, wobei ein Schließen des Verschlusselements vor einem Anheben der Trennwand erfolgt. Das geschlossene Verschlusselement hindert ein in dem Fermentierbehälter vorhandenes und infolge des Anhebens der Trennwand frei bewegliches Biogas an einem unkontrollierten Entweichen durch die Wartungsöffnung nach außen.

Vorteilhaft ist vorgesehen, dass die Trennwand erst von dem Flüssigkeitsspiegel angehoben wird, wenn das Verschlusselement geschlossen ist. Mit anderen Worten ist ausgeschlossen, dass das die Wartungsöffnung freigegeben ist, nachdem die Trennwand von dem Flüssigkeitsspiegel angehoben ist.

In diesem Zusammenhang wird insbesondere darauf hingewiesen, dass alle im Bezug auf die Vorrichtung beschriebenen Merkmale und Eigenschaften aber auch Verfahrensweisen sinngemäß auch bezüglich der Formulierung des erfindungsgemäßen Verfahrens übertragbar und im Sinne der Erfindung einsetzbar und als mitoffenbart gelten. Gleiches gilt auch in umgekehrter Richtung, das bedeutet, nur im Bezug auf das Verfahren genannte, bauliche also vorrichtungsgemäße Merkmale können auch im Rahmen der Vorrichtungsansprüche berücksichtigt und beansprucht werden und zählen ebenfalls zur Offenbarung.

In der Zeichnung ist die Erfindung insbesondere in einem Ausführungsbeispiel schematisch dargestellt. Es zeigen:
- Fig. 1a: in einer Querschnittsdarstellung eine Seitenansicht eine Vorrichtung nach einer Ausführungsform der vorliegenden Erfindung in einer verriegelten Schließstellung des Verschlusselements;
- Fig. 1b: eine Draufsicht der in Fig. 1a gezeigten Vorrichtung;
- Fig. 2: in einer perspektivischen Darstellung eine Ansicht der in Fig. 1a gezeigten Vorrichtung in einer entriegelten Schließstellung des Verschlusselements;
- Fig. 3: in einer perspektivischen Darstellung eine Ansicht der in Fig. 1a gezeigten Vorrichtung in einer Offenstellung des Verschlusselements.

In den Figuren sind gleiche oder einander entsprechende Elemente jeweils mit den gleichen Bezugszeichen bezeichnet und werden daher, sofern nicht zweckmäßig, nicht erneut beschrieben. Die in der gesamten Beschreibung enthaltenen Offenbarungen sind sinngemäß auf gleiche Teile mit gleichen Bezugszeichen bzw. gleichen Bauteilbezeichnungen übertragbar. Auch sind die in der Beschreibung gewählten Lageangaben, wie z.B. oben, unten, seitlich usw. auf die unmittelbar beschriebene sowie dargestellte Figur bezogen und sind bei einer Lageänderung sinngemäß auf die neue Lage zu übertragen. Weiterhin können auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen.

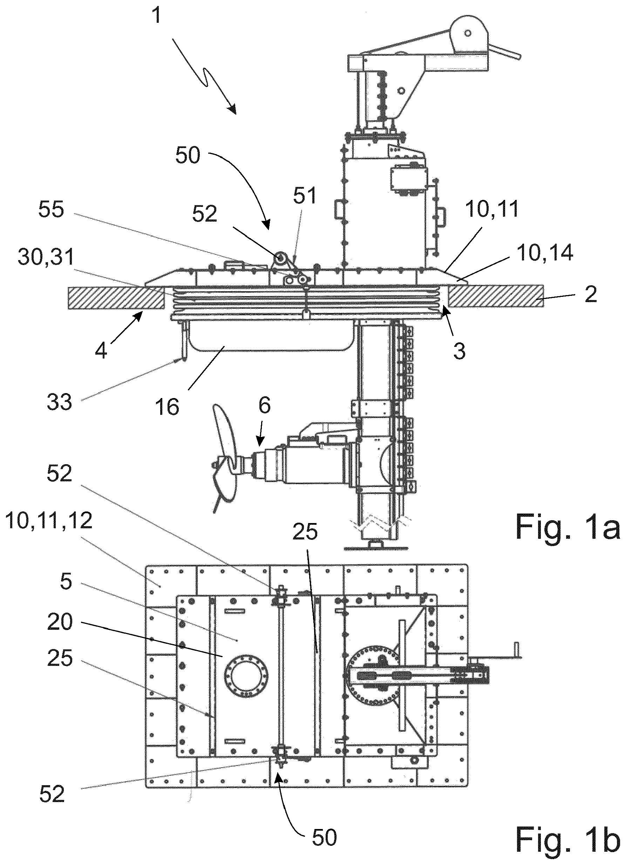

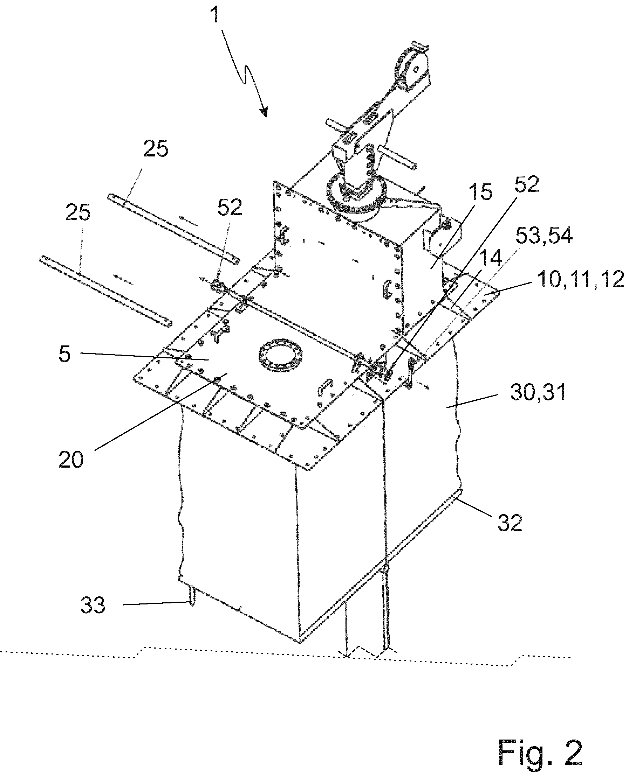

Die Figuren 1a, 1b und 2 zeigen eine Vorrichtung 1 zum Öffnen und Schließen einer in einer Deckwand 2 eines Fermentierbehälters einer Biogasanlage vorgesehenen Wartungsöffnung 3 aus verschiedenen Blickrichtungen und in verschiedenen Zuständen. Die Biogasanlage umfasst eine Rühreinheit 6, die mittels einer Linearführung an einer sich durch die Wartungsöffnung 3 erstreckenden Säule 5 verfahrbar gehalten ist. Zum Verfahren der Rühreinheit 6 kann beispielsweise ein Seilzug mit einer außerhalb des Fermentierbehälters angeordneten Antriebseinheit 53 vorgesehen sein.

Die Vorrichtung 1 ist in die Wartungsöffnung 3 einsetzbar und umfasst einen Körper 10 zum Verbinden der Vorrichtung 1 mit der Wartungsöffnung 3 des Fermentierbehälters und einem Verschlusselement 20 zum wahlweisen Öffnen und Schließen der Wartungsöffnung 3.

Der Körper 10 umfasst einen mit einem Randbereich 4 der Wartungsöffnung 3 verschraubbaren geschlossenen Rahmen 11. Der Rahmen 11 weist eine umlaufende erste Anlagefläche 12 aufweist, die im bestimmungsgemäß montierten Zustand der Vorrichtung 1 an dem Randbereich 4 der Wartungsöffnung 3 anliegt. Ferner weist der Rahmen 11 eine umlaufende zweite Anlagefläche 13 auf, an der das Verschlusselement 20 in einer Schließstellung des Verschlusselements 20 anliegt. Der Rahmen 11 weist zudem eine Mehrzahl von flachen Verbindungsstreben 14 auf, die sich beabstandet zueinander und verteilt entlang des Umfangs des Rahmens 11 jeweils senkrecht zwischen der ersten Anlagefläche 12 und der zweiten Anlagefläche 13 erstrecken und eine trapezförmige Außenkontur aufweisen. Die Vorrichtung 1 umfasst eine Wartungsklappe 16, die an dem Rahmen verschwenkbar gehalten ist. Sie ist nur bei einer geöffneten Wartungsöffnung 3 zugänglich und erlaubt ein Öffnen und Schließen der Wartungsöffnung 3 unabhängig von dem Verschlusselement 20.

Das Verschlusselement 20 ist an der Vorrichtung 1 demontierbar angeordnet und mit dem Körper 10 lösbar verbunden, kann aber auch an dem Körper 10 drehbar gelagert und wischen einer Schließstellung und einer Offenstellung des Verschlusselements 20 verschwenkbar gehalten sein.

Das Verschlusselement 20 umfasst eine aus einem Metall gefertigte Platte 21, die zwei Plattenabschnitte 22, 23 aufweist, die sich senkrecht zueinander erstrecken und an einer gemeinsamen Kante 24 miteinander verbunden sind. In der Schließstellung des Verschlusselements 20 verschließt ein Plattenabschnitt 23 einen kastenförmigen Aufbau 15 des Körpers 10 seitlich.

Das Verschlusselement 20 weist zwei längliche Verstärkungsprofile 25 auf, die mit einem Plattenabschnitt 22 verschraubbar sind und sich im bestimmungsgemäß verschraubten Zustand parallel zueinander und parallel zu dem Plattenabschnitt 22 erstrecken.

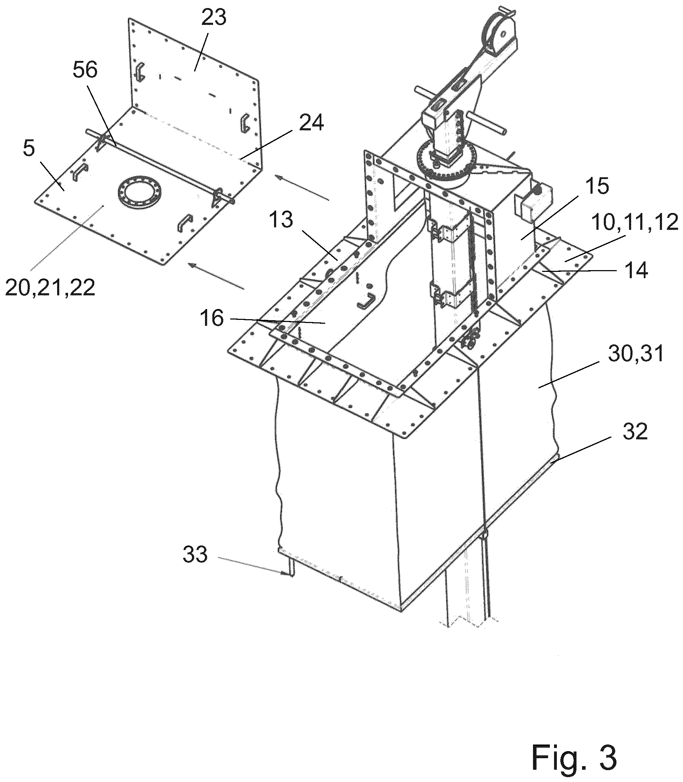

Ferner umfasst die Vorrichtung 1 eine Trennwand 30 zum Abtrennen eines Volumens unterhalb der Wartungsöffnung 3 und einen Betätigungsmechanismus zum wahlweisen Absenken und Anheben der Trennwand 30 wischen einer in Figur 1a gezeigten angehobenen Ruhestellung und einer in den Figuren 2 und 3 gezeigten abgesenkten Trennstellung.

Die Trennwand 30 ist an dem Körper 10 befestigt und zum gasdichten Verbinden eines in dem Inneren des Fermentierbehälters angeordneten Flüssigkeitsspiegels mit dem Körper 10 ausgebildet und umfasst eine schlauchförmige Schürze 31. Sie besteht aus einem aus Kunststoff gefertigten Gewebe, kann aber auch aus einem anderen Material gefertigt sein. Ferner ist die schlauchförmige Schürze 31 ziehharmonikaartig faltbar ausgebildet, kann aber auch mehrere Wandelemente umfassen, die relativ zueinander teleskopartig verschiebbar ausgebildet sind.

Ferner umfasst die Trennwand 30 einen starren Rahmen 32, der an einem dem Körper 10 abgewandten freien Endbereich der Trennwand 30 angeordnet und mit diesem verbunden ist. Die Trennwand 30 weist zudem eine Sonde 33 zum Erfassen eines elektrischen Widerstands auf. Die Sonde 33 ist an einem dem Körper 10 abgewandten Endbereich der Trennwand 30 angeordnet, stabförmig ausgebildet und erstreckt sich von dem Körper 10 weg weisend.

Die Vorrichtung 1 umfasst einen an dem Körper 10 vorgesehenen Verriegelungsmechanismus zum Verriegeln und Entriegeln des Verschlusselements 20 in einer Schließstellung des Verschlusselements 20.

Der Verriegelungsmechanismus ist derart ausgebildet, dass das Verschlusselement 20 nur in der abgesenkten Trennstellung der Trennwand 30 verriegelbar und/oder entriegelbar ist. Dazu umfasst der Verriegelungsmechanismus einen an dem Körper 10 vorgesehenen Durchlass, zwei an dem Verschlusselement 20 vorgesehene Durchlässe, die in der Schließstellung des Verschlusselements 20 sämtlich miteinander fluchten, und eine Welle 56. Die Welle 56 durchgreift die Durchlässe in einem verriegelten Zustand des Verriegelungsmechanismus und ist in einem entriegelten Zustand des Verriegelungsmechanismus aus den Durchlässen herausgezogen.

Der Verriegelungsmechanismus umfasst zwei Seiltrommeln 52 und zwei mit jeweils einer Seiltrommel 52 verbundene Seile 51. Die Seiltrommeln 52 sind mit der Welle 56 verbindbar, so dass die Welle 56 im verriegelten Zustand einen zum Absenken und Anheben der Trennwand 30 vorgesehenen Abschnitt jedes Seils 51 trägt.

Die Vorrichtung 1 umfasst weiterhin einen Betätigungsmechanismus 50. Der Betätigungsmechanismus 50 ist derart ausgebildet und angeordnet, dass die Trennwand 30 auf einer der Trennwand 30 gegenüberliegenden Seite des Körpers 10, also an einer Außenseite des Fermentierbehälters betätigbar ist.

Der Betätigungsmechanismus 50 umfasst die in den beiden Durchlässen drehbar gelagerte Welle 56 und die an gegenüberliegenden freien Enden der Welle 56 angeordneten und jeweils mit der Welle 56 drehfest verbundene Seiltrommeln 52 zum wahlweisen Aufrollen und Abrollen der Seile 51. Ein erstes freies Ende jedes Seils 51 ist mit einer Seiltrommel 52 und ein zweites freies Ende jedes Seils 51 mit einem freien Endbereich der Trennwand 30 verbunden und an gegenüberliegenden Seiten des Rahmens 32 befestigt.

Ferner weist der Betätigungsmechanismus 50 eine Antriebseinheit 53 zum drehenden Antreiben der Welle 56 auf, die eine mit einer Seiltrommel 52 lösbar verbindbare Kurbel 54 in Gestalt einer gebremsten Ratschenkurbel umfasst.

Der Betätigungsmechanismus 50 umfasst weiterhin zu jedem Seil 51 eine an dem Körper 10 drehbar gelagerte Umlenkrolle 55 zum Führen des Seils 51, das sich durch eine in dem Körper 10 vorgesehene Seilöffnung von einer Außenseite der Vorrichtung 1 zu einer Innenseite der Vorrichtung 1 erstreckt.

Im Rahmen einer Betätigung des Verschlusselements 20 erfolgt ein Öffnen des Verschlusselements 20 erst, wenn die Trennwand 30 abgesenkt ist, d.h. wenn die Vorrichtung 1 durch die Trennwand 30 gasdicht mit dem Flüssigkeitsspiegel im Inneren des Fermentierbehälters verbunden ist. Wie in Figur 2 gezeigt, wird zum Entriegeln des Verschlusselements 20 eine Seiltrommel 52 von der Welle 56 gelöst und die Welle 56 aus den Durchlässen seitlich herausgezogen. Zudem werden die beiden Verstärkungsprofile 25 gelöst und entfernt. Dann wird Verschlusselement 20 aus von der Vorrichtung 1 gelöst und aus der Schließstellung in eine Offenstellung bewegt, wie in Figur 3 gezeigt ist.

Weiterhin erfolgt im Rahmen der Betätigung des Verschlusselements 20 ein Schließen des Verschlusselements 20 vor einem Anheben der Trennwand 30, d.h. die Trennwand 30 wird erst von dem Flüssigkeitsspiegel angehoben, wenn das Verschlusselement 20 geschlossen ist.

Ein wesentlicher Vorteil der erfindungsgemäßen Vorrichtung 1 besteht darin, dass die Trennwand ein unkontrolliertes Entweichen eines überwiegenden Anteils von in dem Fermentierbehälter vorhandenem Biogas nach außen zuverlässig verhindert. Durch einen Verriegelungsmechanismus kann die Vorrichtung zudem sicherstellen, dass die Wartungsöffnung nur bei abgesenkter Trennwand verschlossen oder freigegeben wird.

Nachfolgend werden mögliche Merkmale des Vorschlages strukturiert wiedergegeben. Die nachfolgenden strukturiert wiedergegebenen Merkmale können beliebig untereinander kombiniert werden und können in beliebiger Kombination in die Ansprüche der Anmeldung aufgenommen werden. Dem Fachmann ist klar, dass sich die Erfindung bereits aus dem Gegenstand mit den wenigsten Merkmalen ergibt. Insbesondere sind nachfolgend vorteilhafte oder mögliche Ausgestaltungen, nicht jedoch die einzig möglichen Ausgestaltungen der Erfindung wiedergegeben.

### Die Erfindung umfasst:

Eine Vorrichtung zum Öffnen und Schließen einer in einer Deckwand eines Fermentierbehälters vorgesehenen Wartungsöffnung, mit einem Körper zum Verbinden der Vorrichtung mit einer Wartungsöffnung eines Fermentierbehälters und einem Verschlusselement zum wahlweisen Öffnen und Schließen der Wartungsöffnung, einer Trennwand zum Abtrennen eines Volumens unterhalb der Wartungsöffnung und einem Betätigungsmechanismus zum wahlweisen Absenken und Anheben der Trennwand zwischen einer angehobenen Ruhestellung und einer abgesenkten Trennstellung.

Eine Vorrichtung zum Öffnen und Schließen einer in einer Deckwand eines Fermentierbehälters vorgesehenen Wartungsöffnung, mit einer Trennwand zum Abtrennen eines Volumens unterhalb der Wartungsöffnung und einem Betätigungsmechanismus zum wahlweisen Absenken und Anheben der Trennwand zwischen einer angehobenen Ruhestellung und einer abgesenkten Trennstellung.

Eine wie zuvor ausgeführte Vorrichtung zum Öffnen und Schließen einer in einer Deckwand eines Fermentierbehälters vorgesehenen Wartungsöffnung, wobei die Trennwand zum gasdichten Verbinden eines in dem Inneren des Fermentierbehälters angeordneten Flüssigkeitsspiegels mit dem Körper ausgebildet ist.

Eine wie zuvor ausgeführte erfindungsgemäße Vorrichtung zum Öffnen und Schließen einer in einer Deckwand eines Fermentierbehälters vorgesehenen Wartungsöffnung, umfassend einen an der Vorrichtung, insbesondere an dem Körper vorgesehenen Verriegelungsmechanismus zum Verriegeln und Entriegeln des Verschlusselements in einer Schließstellung des Verschlusselements.

Eine wie zuvor ausgeführte erfindungsgemäße Vorrichtung zum Öffnen und Schließen einer in einer Deckwand eines Fermentierbehälters vorgesehenen Wartungsöffnung, wobei der Verriegelungsmechanismus derart ausgebildet ist, dass das Verschlusselement nur in der abgesenkten Trennstellung der Trennwand verriegelbar und/oder entriegelbar ist.

Eine wie zuvor ausgeführte Vorrichtung zum Öffnen und Schließen einer in einer Deckwand eines Fermentierbehälters vorgesehenen Wartungsöffnung, wobei der Verriegelungsmechanismus einen an dem Körper vorgesehenen Durchlass, einen an dem Verschlusselement vorgesehenen Durchlass, die in der Schließstellung des Verschlusselements miteinander fluchten, und eine Welle umfasst, wobei die Welle die beiden Durchlässe in einem verriegelten Zustand des Verriegelungsmechanismus durchgreift und in einem entriegelten Zustand des Verriegelungsmechanismus aus den beiden Durchlässen herausgezogen ist.

Eine wie zuvor ausgeführte Vorrichtung zum Öffnen und Schließen einer in einer Deckwand eines Fermentierbehälters vorgesehenen Wartungsöffnung, wobei der Verriegelungsmechanismus eine Seiltrommel und ein mit der Seiltrommel verbundenes Seil umfasst.

Eine wie zuvor ausgeführte Vorrichtung zum Öffnen und Schließen einer in einer Deckwand eines Fermentierbehälters vorgesehenen Wartungsöffnung, wobei die Welle im verriegelten Zustand einen zum Absenken und Anheben der Trennwand vorgesehenen Abschnitt des Seils trägt.

Eine wie zuvor ausgeführte Vorrichtung zum Öffnen und Schließen einer in einer Deckwand eines Fermentierbehälters vorgesehenen Wartungsöffnung, wobei der Betätigungsmechanismus derart ausgebildet und angeordnet ist, dass die Trennwand auf einer der Trennwand gegenüberliegenden Seite des Körpers betätigbar ist.

Eine wie zuvor ausgeführte Vorrichtung zum Öffnen und Schließen einer in einer Deckwand eines Fermentierbehälters vorgesehenen Wartungsöffnung, wobei der Betätigungsmechanismus die in den beiden Durchlässen drehbar gelagerte Welle und die mit der Welle drehfest verbundene Seiltrommel zum wahlweisen Aufrollen und Abrollen des Seils umfasst.

Eine wie zuvor ausgeführte Vorrichtung zum Öffnen und Schließen einer in einer Deckwand eines Fermentierbehälters vorgesehenen Wartungsöffnung, wobei ein erstes freies Ende des Seils mit der Seiltrommel und ein zweites freies Ende des Seils mit einem freien Endbereich der Trennwand verbunden sind.

Eine wie zuvor ausgeführte Vorrichtung zum Öffnen und Schließen einer in einer Deckwand eines Fermentierbehälters vorgesehenen Wartungsöffnung, wobei der Betätigungsmechanismus und/oder der Verriegelungsmechanismus zwei Seiltrommeln, die an gegenüberliegenden freien Enden der Welle angeordnet sind, und zwei Seile umfasst, die mit gegenüberliegenden freien Endbereichen der Trennwand verbunden sind.

Eine wie zuvor ausgeführte Vorrichtung zum Öffnen und Schließen einer in einer Deckwand eines Fermentierbehälters vorgesehenen Wartungsöffnung, wobei der Betätigungsmechanismus eine Antriebseinheit zum drehenden Antreiben der Welle aufweist.

Eine wie zuvor ausgeführte Vorrichtung zum Öffnen und Schließen einer in einer Deckwand eines Fermentierbehälters vorgesehenen Wartungsöffnung, wobei die Antriebseinheit eine mit der Seiltrommel lösbar verbindbare Kurbel, insbesondere eine gebremste Ratschenkurbel umfasst.

Eine wie zuvor ausgeführte Vorrichtung zum Öffnen und Schließen einer in einer Deckwand eines Fermentierbehälters vorgesehenen Wartungsöffnung, wobei der Betätigungsmechanismus zu einem, insbesondere zu jedem Seil eine an dem Körper drehbar gelagerte Umlenkrolle zum Führen des Seils umfasst.

Eine wie zuvor ausgeführte Vorrichtung zum Öffnen und Schließen einer in einer Deckwand eines Fermentierbehälters vorgesehenen Wartungsöffnung, wobei sich jedes Seil durch eine in dem Körper vorgesehene Seilöffnung erstreckt.

Eine wie zuvor ausgeführte Vorrichtung zum Öffnen und Schließen einer in einer Deckwand eines Fermentierbehälters vorgesehenen Wartungsöffnung, wobei die Trennwand einen Kunststoff umfasst oder aus einem Kunststoff besteht.

Eine wie zuvor ausgeführte Vorrichtung zum Öffnen und Schließen einer in einer Deckwand eines Fermentierbehälters vorgesehenen Wartungsöffnung, wobei die Trennwand ein Gewebe umfasst oder aus einen Gewebe besteht.

Eine wie zuvor ausgeführte Vorrichtung zum Öffnen und Schließen einer in einer Deckwand eines Fermentierbehälters vorgesehenen Wartungsöffnung, wobei die Trennwand eine schlauchförmige Schürze umfasst.

Eine wie zuvor ausgeführte Vorrichtung zum Öffnen und Schließen einer in einer Deckwand eines Fermentierbehälters vorgesehenen Wartungsöffnung, wobei die Trennwand bzw. die Schürze ziehharmonikaartig faltbar ausgebildet ist.

Eine wie zuvor ausgeführte Vorrichtung zum Öffnen und Schließen einer in einer Deckwand eines Fermentierbehälters vorgesehenen Wartungsöffnung, wobei die Trennwand einen starren Rahmen umfasst, der insbesondere an einem freien Endbereich der Trennwand angeordnet und mit diesem verbunden ist.

Eine wie zuvor ausgeführte Vorrichtung zum Öffnen und Schließen einer in einer Deckwand eines Fermentierbehälters vorgesehenen Wartungsöffnung, wobei der Rahmen an einem dem Körper zugewandten Endbereich und/oder an einem dem Körper abgewandten Endbereich der Trennwand angeordnet ist.

Eine wie zuvor ausgeführte Vorrichtung zum Öffnen und Schließen einer in einer Deckwand eines Fermentierbehälters vorgesehenen Wartungsöffnung, wobei die Trennwand mehrere Wandelemente umfasst.

Eine wie zuvor ausgeführte Vorrichtung zum Öffnen und Schließen einer in einer Deckwand eines Fermentierbehälters vorgesehenen Wartungsöffnung, wobei die Wandelemente relativ zueinander teleskopartig verschiebbar ausgebildet und angeordnet sind.

Eine wie zuvor ausgeführte Vorrichtung zum Öffnen und Schließen einer in einer Deckwand eines Fermentierbehälters vorgesehenen Wartungsöffnung, wobei die Trennwand an dem Körper befestigt ist.

Eine wie zuvor ausgeführte Vorrichtung zum Öffnen und Schließen einer in einer Deckwand eines Fermentierbehälters vorgesehenen Wartungsöffnung, wobei die Trennwand eine Sonde zum Erfassen eines elektrischen Widerstands aufweist.

Eine wie zuvor ausgeführte Vorrichtung zum Öffnen und Schließen einer in einer Deckwand eines Fermentierbehälters vorgesehenen Wartungsöffnung, wobei die Sonde an einem dem Körper abgewandten Endbereich der Trennwand angeordnet ist und/oder stabförmig ausgebildet ist und sich insbesondere von dem Körper weg weisend erstreckt.

Eine wie zuvor ausgeführte Vorrichtung zum Öffnen und Schließen einer in einer Deckwand eines Fermentierbehälters vorgesehenen Wartungsöffnung, wobei das Verschlusselement eine Platte umfasst.

Eine wie zuvor ausgeführte Vorrichtung zum Öffnen und Schließen einer in einer Deckwand eines Fermentierbehälters vorgesehenen Wartungsöffnung, wobei die Platte zwei Plattenabschnitte aufweist, die sich insbesondere senkrecht zueinander erstrecken und/oder an einer gemeinsamen Kante miteinander verbunden sind.

Eine wie zuvor ausgeführte Vorrichtung zum Öffnen und Schließen einer in einer Deckwand eines Fermentierbehälters vorgesehenen Wartungsöffnung, wobei in einer Schließstellung des Verschlusselements ein Plattenabschnitt einen kastenförmigen Aufbau des Körpers seitlich verschließt.

Eine wie zuvor ausgeführte Vorrichtung zum Öffnen und Schließen einer in einer Deckwand eines Fermentierbehälters vorgesehenen Wartungsöffnung, wobei das Verschlusselement ein längliches Verstärkungsprofil aufweist, das mit einem Plattenabschnitt lösbar verbunden, insbesondere verschraubt ist.

Eine wie zuvor ausgeführte Vorrichtung zum Öffnen und Schließen einer in einer Deckwand eines Fermentierbehälters vorgesehenen Wartungsöffnung, wobei das Verschlusselement zwei oder mehr als zwei Verstärkungsprofile aufweist, die sich insbesondere parallel zueinander und/oder jeweils parallel zu dem Plattenabschnitt erstrecken.

Eine wie zuvor ausgeführte Vorrichtung zum Öffnen und Schließen einer in einer Deckwand eines Fermentierbehälters vorgesehenen Wartungsöffnung, wobei das Verschlusselement an der Vorrichtung demontierbar oder drehbar gelagert angeordnet ist.

Eine wie zuvor ausgeführte Vorrichtung zum Öffnen und Schließen einer in einer Deckwand eines Fermentierbehälters vorgesehenen Wartungsöffnung, wobei das Verschlusselement mit dem Körper lösbar verbunden ist oder an dem Körper zwischen einer Schließstellung und einer Offenstellung des Verschlusselements verschwenkbar gehalten ist.

Eine wie zuvor ausgeführte Vorrichtung zum Öffnen und Schließen einer in einer Deckwand eines Fermentierbehälters vorgesehenen Wartungsöffnung, wobei die Vorrichtung in die Wartungsöffnung einsetzbar ist.

Eine wie zuvor ausgeführte Vorrichtung zum Öffnen und Schließen einer in einer Deckwand eines Fermentierbehälters vorgesehenen Wartungsöffnung, wobei der Körper einen mit einem Randbereich der Wartungsöffnung verbindbaren, insbesondere verschraubbaren geschlossenen Rahmen umfasst.

Eine wie zuvor ausgeführte Vorrichtung zum Öffnen und Schließen einer in einer Deckwand eines Fermentierbehälters vorgesehenen Wartungsöffnung, wobei der Rahmen eine umlaufende erste Anlagefläche aufweist, die im bestimmungsgemäß montierten Zustand der Vorrichtung an dem Randbereich der Wartungsöffnung anliegt.

Eine wie zuvor ausgeführte Vorrichtung zum Öffnen und Schließen einer in einer Deckwand eines Fermentierbehälters vorgesehenen Wartungsöffnung, wobei der Rahmen eine umlaufende zweite Anlagefläche aufweist, an der das Verschlusselement in einer Schließstellung des Verschlusselements anliegt.

Eine wie zuvor ausgeführte Vorrichtung zum Öffnen und Schließen einer in einer Deckwand eines Fermentierbehälters vorgesehenen Wartungsöffnung, wobei der Rahmen eine Mehrzahl von flachen Verbindungsstreben aufweist, die sich insbesondere senkrecht zwischen der ersten Anlagefläche und der zweiten Anlagefläche erstrecken und/oder eine trapezförmige Außenkontur aufweisen.

### Die Erfindung umfasst des Weiteren:

Eine Biogasanlage, umfassend einen Fermentierbehälter mit einer in einer Deckwand des Fermentierbehälters ausgebildeten Wartungsöffnung und eine erfindungsgemäße Vorrichtung und/oder eine Vorrichtung, die in einem erfindungsgemäßen Verfahren bedient wird.

Eine Biogasanlage, umfassend eine erfindungsgemäße Vorrichtung und/oder eine Vorrichtung, die in einem erfindungsgemäßen Verfahren bedient wird.

Eine wie zuvor ausgeführte Biogasanlage, wobei die Biogasanlage eine Rühreinheit umfasst.

### Die Erfindung umfasst ferner:

Ein Verfahren zum Öffnen eines Verschlusselements für einen Fermentierbehälter einer erfindungsgemäßen Biogasanlage, wobei ein Öffnen des Verschlusselements erst erfolgt, wenn die Trennwand abgesenkt ist.

Das zuvor genannte Verfahren zum Öffnen eines Verschlusselements für einen Fermentierbehälter einer Biogasanlage, wobei ein Öffnen des Verschlusselements erst erfolgt, wenn die Vorrichtung durch die Trennwand gasdicht mit dem Flüssigkeitsspiegel im Inneren des Fermentierbehälters verbunden ist.

### Weiterhin umfasst die Erfindung:

Ein Verfahren zum Schließen eines Verschlusselements für einen Fermentierbehälter einer erfindungsgemäßen Biogasanlage, wobei ein Schließen des Verschlusselements vor einem Anheben der Trennwand erfolgt.

Das zuvor genannte Verfahren zum Schließen eines Verschlusselements für einen Fermentierbehälter einer Biogasanlage, wobei die Trennwand erst von dem Flüssigkeitsspiegel angehoben wird, wenn das Verschlusselement geschlossen ist.

Die jetzt mit der Anmeldung und später eingereichten Ansprüche sind ohne Präjudiz für die Erzielung weitergehenden Schutzes.

Sollte sich hier bei näherer Prüfung, insbesondere auch des einschlägigen Standes der Technik, ergeben, dass das eine oder andere Merkmal für das Ziel der Erfindung zwar günstig, nicht aber entscheidend wichtig ist, so wird selbstverständlich schon jetzt eine Formulierung angestrebt, die ein solches Merkmal, insbesondere im Hauptanspruch, nicht mehr aufweist. Auch eine solche Unterkombination ist von der Offenbarung dieser Anmeldung abgedeckt.

Es ist weiter zu beachten, dass die in den verschiedenen Ausführungsformen beschriebenen und in den Figuren gezeigten Ausgestaltungen und Varianten der Erfindung beliebig untereinander kombinierbar sind. Dabei sind einzelne oder mehrere Merkmale beliebig gegeneinander austauschbar. Diese Merkmalskombinationen sind ebenso mit offenbart.

Die in den abhängigen Ansprüchen angeführten Rückbeziehungen weisen auf die weitere Ausbildung des Gegenstandes des Hauptanspruches durch die Merkmale des jeweiligen Unteranspruches hin. Jedoch sind diese nicht als ein Verzicht auf die Erzielung eines selbständigen, gegenständlichen Schutzes für die Merkmale der rückbezogenen Unteransprüche zu verstehen.

Merkmale, die nur in der Beschreibung offenbart wurden oder auch Einzelmerkmale aus Ansprüchen, die eine Mehrzahl von Merkmalen umfassen, können jederzeit als von erfindungswesentlicher Bedeutung zur Abgrenzung vom Stande der Technik in den oder die unabhängigen Anspruch/Ansprüche übernommen werden, und zwar auch dann, wenn solche Merkmale im Zusammenhang mit anderen Merkmalen erwähnt wurden beziehungsweise im Zusammenhang mit anderen Merkmalen besonders günstige Ergebnisse erreichen.

## Patentansprüche

1. Vorrichtung zum Öffnen und Schließen einer in einer Deckwand (2) eines Fermentierbehälters vorgesehenen Wartungsöffnung (3), mit einem Körper (10) zum Verbinden der Vorrichtung (1) mit einer Wartungsöffnung (3) eines Fermentierbehälters und einem Verschlusselement (20) zum wahlweisen Öffnen und Schließen der Wartungsöffnung (3), wobei eine Trennwand (30) zum gasdichten Abtrennen eines Volumens unterhalb der Wartungsöffnung (3) und einen Betätigungsmechanismus (50) zum wahlweisen Absenken und Anheben der Trennwand (30) zwischen einer angehobenen Ruhestellung und einer abgesenkten Trennstellung vorgesehen sind **gekennzeichnet durch** einen an der Vorrichtung (1), insbesondere an dem Körper (10) vorgesehenen Verriegelungsmechanismus zum Verriegeln und Entriegeln des Verschlusselements (20) in einer Schließstellung des Verschlusselements (20) wobei der Verriegelungsmechanismus derart ausgebildet ist, dass das Verschlusselement (20) nur in der abgesenkten Trennstellung der Trennwand (30) verriegelbar und/oder entriegelbar ist.

2. Vorrichtung nach Anspruch 1, **dadurch gekennzeichnet, dass** die Trennwand (30) zum gasdichten Verbinden eines in dem Inneren des Fermentierbehälters angeordneten Flüssigkeitsspiegels mit dem Körper (10) ausgebildet ist und die Trennwand (30) eine schlauchförmige Schürze (31) umfasst.

3. Vorrichtung nach einem der vorhergehenden Ansprüche, **dadurch gekennzeichnet, dass** die Trennwand (30) einen Kunststoff umfasst oder aus einem Kunststoff besteht und/oder die Trennwand (30) ein Gewebe umfasst oder aus einem Gewebe besteht und/oder die Trennwand (30), bzw. die Schürze (31) ziehharmonikaartig faltbar ausgebildet ist und/oder die Trennwand (30) einen starren Rahmen (32) umfasst, der insbesondere an einem freien Endbereich der Trennwand (30) angeordnet und mit diesem verbunden ist und/oder die Trennwand (30) mehrere Wandelemente umfasst und/oder die Trennwand (30) an dem Körper (10) befestigt ist und/oder die Trennwand (30) eine Sonde (33), insbesondere zum Erfassen eines elektrischen Widerstands aufweist.

4. Vorrichtung nach einem der vorhergehenden Ansprüche, **dadurch gekennzeichnet, dass** der Verriegelungsmechanismus einen an dem Körper (10) vorgesehenen Durchlass, einen an dem Verschlusselement (20) vorgesehenen Durchlass, die in der Schließstellung des Verschlusselements (20) miteinander fluchten, und eine Welle (56) umfasst, wobei die Welle (56) die beiden Durchlässe in einem verriegelten Zustand des Verriegelungsmechanismus durchgreift und in einem entriegelten Zustand des Verriegelungsmechanismus aus den beiden Durchlässen herausgezogen ist.

5. Vorrichtung nach einem der vorhergehenden Ansprüche, **dadurch gekennzeichnet, dass** der Verriegelungsmechanismus eine Seiltrommel (52) und ein mit der Seiltrommel (52) verbundenes Seil (51) umfasst.

6. Vorrichtung nach einem der vorhergehenden Ansprüche, **dadurch gekennzeichnet, dass** der Betätigungsmechanismus (50) derart ausgebildet und angeordnet ist, dass die Trennwand (30) auf einer der Trennwand (30) gegenüberliegenden Seite des Körpers (10) betätigbar ist und/oder der Betätigungsmechanismus (50) die in den beiden Durchlässen drehbar gelagerte Welle (56) und die mit der Welle (56) drehfest verbundene Seiltrommel (52) zum wahlweisen Aufrollen und Abrollen des Seils (51) umfasst.

7. Vorrichtung nach einem der vorhergehenden Ansprüche, **dadurch gekennzeichnet, dass** der Betätigungsmechanismus (50) und/oder der Verriegelungsmechanismus zwei Seiltrommeln (52), die an gegenüberliegenden freien Enden der Welle (56) angeordnet sind und zwei Seile (51) umfasst, die mit gegenüberliegenden freien Endbereichen der Trennwand (30) verbunden sind und/oder der Betätigungsmechanismus (50) eine Antriebseinheit (53) zum drehenden Antreiben der Welle (56) aufweist und/oder der Betätigungsmechanismus (50) zu einem, insbesondere zu jedem Seil (51) eine an dem Körper (10) drehbar gelagerte Umlenkrolle (55) zum Führen des Seils (51) umfasst.

8. Vorrichtung nach einem der vorhergehenden Ansprüche, **dadurch gekennzeichnet, dass** das Verschlusselement (20) eine Platte (21) umfasst und/oder das Verschlusselement (20), insbesondere die Platte (21) zwei Plattenabschnitte (22, 23) aufweist, die sich insbesondere senkrecht zueinander erstrecken und/oder an einer gemeinsamen Kante (24) miteinander verbunden sind und/oder in der Schließstellung des Verschlusselements (20) ein Plattenabschnitt (23) einen kastenförmigen Aufbau (15) des Körpers (10) seitlich verschließt und/oder das Verschlusselement (20) ein längliches Verstärkungsprofil (25) aufweist, das mit einem Plattenabschnitt (22, 23) lösbar verbunden, insbesondere verschraubt ist und/oder das Verschlusselement (20) zwei oder mehr als zwei Verstärkungsprofile (25) aufweist, die sich insbesondere parallel zueinander und/oder jeweils parallel zu dem Plattenabschnitt (22, 23) erstrecken.

9. Vorrichtung nach einem der vorhergehenden Ansprüche, **dadurch gekennzeichnet, dass** das Verschlusselement (20) an der Vorrichtung (1) demontierbar oder drehbar gelagert angeordnet ist und/oder das Verschlusselement (20) mit dem Körper (10) lösbar verbunden ist oder an dem Körper (10) zwischen der Schließstellung und einer Offenstellung des Verschlusselements (20) verschwenkbar gehalten ist.

10. Vorrichtung nach einem der vorhergehenden Ansprüche, **dadurch gekennzeichnet, dass** die Vorrichtung (1) in die Wartungsöffnung (3) einsetzbar ist und/oder die Welle (56) im verriegelten Zustand einen zum Absenken und Anheben der Trennwand (30) vorgesehenen Abschnitt des Seils (51) trägt und/oder ein erstes freies Ende des Seils (51) mit der Seiltrommel (52) und ein zweites freies Ende des Seils (51) mit einem freien Endbereich der Trennwand (30) verbunden sind und/oder die Antriebseinheit (53) eine mit der Seiltrommel (52) lösbar verbindbare Kurbel (54), insbesondere eine gebremste Ratschenkurbel umfasst und/oder sich jedes Seil (51) durch eine in dem Körper (10) vorgesehene Seilöffnung erstreckt und/oder der Rahmen (32) an einem dem Körper (10) zugewandten Endbereich und/oder an einem dem Körper (10) abgewandten Endbereich der Trennwand (30) angeordnet ist und/oder die Wandelemente relativ zueinander teleskopartig verschiebbar ausgebildet und angeordnet sind und/oder die Sonde (33) an einem dem Körper (10) abgewandten Endbereich der Trennwand (30) angeordnet ist und/oder stabförmig ausgebildet ist und sich insbesondere von dem Körper (10) weg weisend erstreckt.

11. Vorrichtung nach einem der vorhergehenden Ansprüche, **dadurch gekennzeichnet, dass** der Körper (10) einen mit einem Randbereich (4) der Wartungsöffnung (3) verbindbaren, insbesondere verschraubbaren geschlossenen Rahmen (11) umfasst, wobei insbesondere der Rahmen (11) eine umlaufende erste Anlagefläche (12) aufweist, die im bestimmungsgemäß montierten Zustand der Vorrichtung (1) an dem Randbereich (4) der Wartungsöffnung (3) anliegt.

12. Vorrichtung nach einem der vorhergehenden Ansprüche, **dadurch gekennzeichnet, dass** der Rahmen (11) eine umlaufende zweite Anlagefläche (13) aufweist, an der das Verschlusselement (20) in der Schließstellung des Verschlusselements (20) anliegt und/oder der Rahmen (11) eine Mehrzahl von flachen Verbindungsstreben (14) aufweist, die sich insbesondere senkrecht zwischen der ersten Anlagefläche (12) und der zweiten Anlagefläche (13) erstrecken und/oder eine trapezförmige Außenkontur aufweisen.

13. Biogasanlage, umfassend einen Fermentierbehälter mit einer in einer Deckwand (2) des Fermentierbehälters ausgebildeten Wartungsöffnung (3), **gekennzeichnet durch** eine Vorrichtung (1) nach einem der vorhergehenden Ansprüche und/oder eine Vorrichtung (1), die in einem Verfahren nach einem der Ansprüche 14 bis 15 bedient wird, und die Biogasanlage insbesondere eine Rühreinheit (6) umfasst.

14. Verfahren zum Öffnen eines Verschlusselements (20) für einen Fermentierbehälter einer Biogasanlage nach Anspruch 13, wobei ein Öffnen des Verschlusselements (20) erst erfolgt, wenn die Trennwand (30) abgesenkt ist.

15. Verfahren nach Anspruch 14, **dadurch gekennzeichnet, dass** ein Öffnen des Verschlusselements (20) erst erfolgt, wenn die Vorrichtung (1) durch die Trennwand (30) gasdicht mit dem Flüssigkeitsspiegel im Inneren des Fermentierbehälters verbunden ist und/oder ein Schließen des Verschlusselements (20) vor einem Anheben der Trennwand (30) erfolgt und/oder die Trennwand (30) erst von dem Flüssigkeitsspiegel angehoben wird, wenn das Verschlusselement (20) geschlossen ist.
